# EUROPEAN PATENT APPLICATION

(11) **EP 4 357 943 A1**
(43) Date of publication of application: **24.04.2024**
(21) Application number: 22202408.5
(22) Date of filing: 19.10.2022
(51) Int. Cl.: G06F 17/18, G06N 3/00

(54) **GENERATING SIMULATED POPULATION DATASET**

(71) Applicant: Koninklijke Philips N.V., 5656 AG Eindhoven (NL)
(72) Inventor: DE VRIES, Jan Johannes Gerardus, Eindhoven (NL)
(74) Representative: Philips Intellectual Property & Standards

(57) **Abstract**

A mechanism for simulating a dataset for a population of medical subjects. Statistical information about an example population dataset is used to control the generation of a simulated population dataset, such that the statistical properties of the simulated population dataset are similar to those of the example population dataset.

## Description

### FIELD OF THE INVENTION

The present invention relates to the field of simulating a population dataset.

### BACKGROUND OF THE INVENTION

There is an ever increasing interest in the use of machine-learning algorithms, such as neural networks, to perform any number of predictive tasks. They find particular use in the medical field to analyze or assess the characteristics of a medical subject to aid in the identification of a pathology and/or diagnosis.

Typically, in order to accurately train a machine-learning algorithm, a large amount of ground truth data is required. In the field of medical science, this ground truth data often takes the form of a population dataset, which contains values for a plurality of subject parameters for a plurality of medical subjects.

However, the use and availability of such population datasets is becoming increasingly difficult, due to ever increasing legal and moral restrictions to maintain privacy of the medical subjects. In particular, recent trends have required (re)consent and/or exempting justification to be provided in order to permit analysis and/or processing of a population dataset. This creates a significant burden on a data analyst, and can provide extremely difficult, especially for retrospective data analysis.

It would therefore be desirable to provide population datasets that are not restricted or constrained by any such restrictions, whilst still providing adequate data to determine inter-variable relationships that resemble ground-truth inter-variable relationships.

Whilst population datasets find particular use in the training of machine-learning algorithms, it will be appreciated that there are other advantages to population datasets, e.g., for data science, research or analysis purposes.

### SUMMARY OF THE INVENTION

The invention is defined by the claims.

According to examples in accordance with an aspect of the invention, there is provided a computer-implemented method for generating a simulated population dataset.

The computer-implemented method comprises: obtaining one or more first values, each first value being a value of a statistical property of an example population dataset, the example population dataset containing example values for a plurality of subject parameters for a plurality of example medical subjects; defining a simulated population dataset containing simulated values for the plurality of subject parameters for a plurality of simulated medical subjects; and performing an iterative process comprising: modifying at least some of the simulated values in the simulated population dataset; and determining one or more second values by processing the simulated population dataset using at least one statistical analysis technique, each second value being the value of a statistical property of the simulated population dataset, wherein each second value represents a value of a same statistical property as a corresponding first value.

The iterative process is terminated responsive to a similarity between the one or more first values and the one or more second values meeting one or more predetermined criteria.

*The present disclosure provides a mechanism for simulating a population dataset of (simulated) medical subjects, which can be used for training a machine-learning method or other advantageous use-case scenarios (such as data analysis or data science). In particular, it is noted that the simulated population dataset is suitable for use in training a machine-learning method.*

*One particular advantage of simulating a population dataset is that, by definition, any entry or value in the population dataset cannot be attributed to a specific individual, as the values are simulated. This avoids any legal and privacy issues (e.g., mandatory anonymity) involved in handling a large amount of patient data.*

*Another advantage of simulating a population dataset is that additional data is generated, beyond that previously available, for training and*/*or analysis purposes.*

*The simulated population dataset is generated in a manner such that the statistical properties of the simulated population dataset match or resemble those of an example population dataset (i.e., a "true" or "real" population dataset containing information about real medical subjects). This approach has been identified as generating sufficiently similar or accurate data for high levels of accuracy of later analysis or training (e.g., of a machine-learning model).*

*The example population dataset may contain a plurality of first data entries, each first data entry representing a different example medical subject and comprising a plurality of first data fields that each store a value for a different one of a plurality of subject parameters. The simulated population dataset may similarly contain a plurality of second data entries, each second data entry representing a different simulated medical subject and comprising a plurality of second data fields that each store a value for a different one of the plurality of subject parameters. It will be appreciated that the number of second data fields is not greater than the number of first data fields, e.g., they may be the same.*

In some examples, the one or more first values comprises a co-variance matrix of the example population dataset; and the one or more second values comprises a co-variance matrix of the simulated population dataset.

*Use of a co-variance matrix ensures that the relationship between different statistical properties in the example population dataset is preserved in the simulated population dataset. This improves an accuracy of training a later machine-learning model (using the simulated population dataset as ground-truth data) to perform a predictive function, as well as ensuring that any data analysis of the simulated population dataset, e.g., to identify trends or relationships, is accurate and representative of the "real-life*" *or ground truth scenario.*

The step of defining a simulated population dataset may comprise generating a random or pseudorandom value for each of the simulated values. *This provides a simple and intuitive mechanism for setting the initial values of the simulated population dataset, whilst also reducing a likelihood of a local minimum being reached during iterative modifications.*

*In some examples, the step of generating a random or pseudorandom value for each of the simulated values is configured such that the distribution of values throughout the simulated population dataset is uniform.*

*In some examples,* the example population dataset and the simulated population dataset are both normalized datasets.

In some examples, the example population dataset is produced by performing a normalization function on an initial example population dataset; and the computer-implemented method further comprises, after the iterative process is terminated, performing the inverse of the normalization function on the simulated population dataset.

*This approach ensures that the values simulated population dataset matches or resembles expected values for the subject parameters.*

The plurality of subject parameters may comprise at least one binary parameter and/or at least one categorical parameter.

The step of modifying at least some of the simulated values may comprises: selecting a plurality of simulated values using a random or pseudorandom selection process; and modifying the selected values.

The step of modifying at least some of the simulated values may comprise performing larger modifications to the at least some simulated values during temporally earlier iterations of the iterative process than during temporally later iterations of the iterative process.

In some examples, the iterative process further comprises: processing the one or more first values and the one or more second values to determine a measure of similarity between the one or more first values and the one or more second values. *The measure of similarity may be used as the similarity between the one or more first values and the one or more second values to control the termination of the iterative process. A measure of difference is functionally equivalent to a measure of similarity, in that they both represent or change responsive to a similarity between the two data sets.*

*A measure of similarity is a value that changes responsive to a similarity and*/*or difference between the first and second value(s) changing. In particular, the greater the measure of similarity, the greater the similarity. It will be appreciated that a measure of similarity can also or otherwise act as a measure of difference (and vice versa).*

*The predetermined similarity value may, for instance, be a value set or defined by a user or individual.*

The method may comprise, responsive to the measure of similarity meeting predetermined threshold conditions, accepting the modification to the at least some of the simulated values in the simulated population dataset; and responsive to the measure of similarity failing to meet the predetermined threshold conditions, discarding or rejecting the modification to the at least some of the simulated values in the simulated population dataset.

In some examples, the one or more first values comprises a measure of skewness and/or a measure of kurtosis of the example population dataset; and the one or more second values comprises a measure of skewness and/or a measure of kurtosis of the simulated population dataset.

The one or more predetermined criteria may include a criterion that a measure of similarity between the one or more first values and the one or more second values meets predetermined threshold conditions indicating that the one or more first values and the one or more second values are similar to one another.. *As previously explained, a measure of similarity can also or otherwise act as a measure of difference (and vice versa).*

The step of obtaining one or more first values may comprise: obtaining the example population dataset; and processing the example population dataset using the at least one statistical analysis technique to produce the one or more first values. *Thus, the example population dataset may be obtained and processed to produce the first value(s).*

There is also proposed a computer-implemented method for training a machine-learning method to perform a predictive task. The computer-implemented method comprises: obtaining a population dataset comprising a simulated population dataset generated by performing any previously described method; and training the machine-learning method to perform the predictive task using the population dataset as ground truth data.

There is also proposed a computer program product comprising computer program code means which, when executed on a computing device having a processing system, cause the processing system to perform all of the steps of any herein disclosed method. There is also proposed a non-transitory storage medium that stores or comprises the computer program code.

There is also proposed a processing system for generating a simulated population dataset.

The processing system is configured to: obtain one or more first values, each first value being a value of a statistical property of an example population dataset, the example population dataset containing example values for a plurality of subject parameters for a plurality of example medical subjects; define a simulated population dataset containing simulated values for the plurality of subject parameters for a plurality of simulated medical subjects; and perform an iterative process.

The iterative process comprises: modifying at least some of the simulated values in the simulated population dataset; and determining one or more second values by processing the simulated population dataset, each second value being the value of a statistical property of the simulated population dataset, wherein each second value represents a value of a same statistical property as a corresponding first value.

The iterative process is terminated responsive to a similarity between the one or more first values and the one or more second values meeting one or more predetermined criteria.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flowchart illustrating a method according to an embodiment; and
Fig. 2 is a flowchart illustrating a method according to a further embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for simulating a dataset for a population of medical subjects. Statistical information about an example population dataset is used to control the generation of a simulated population dataset, such that the statistical properties of the simulated population dataset are similar to those of the example population dataset.

Embodiments are based on the realization that a population dataset can be simulated that exhibits sufficient similarity to a true population dataset if statistical information of a true (e.g., real or ground truth) population dataset is used to control the generation of the simulated dataset. This means that a population dataset can be produced for use in data analysis and/or training of a machine-learning algorithm without needing to consider privacy and/or anonymity matters. The proposed approach also produces additional data that can be used, e.g., in addition to the real population dataset.

Disclosed approaches can be used to produce a simulated population dataset for any suitable purpose, including training of a machine-learning algorithm (e.g., for performing analysis and/or diagnosis of a medical subject) or for data analysis purposes.

In the context of the present disclosure, the term "randomized" is used to refer to both a true random value and a pseudo-random value. Approaches for generating a random or pseudo-random value are well-established in the art, and are not disclosed for the sake of conciseness.

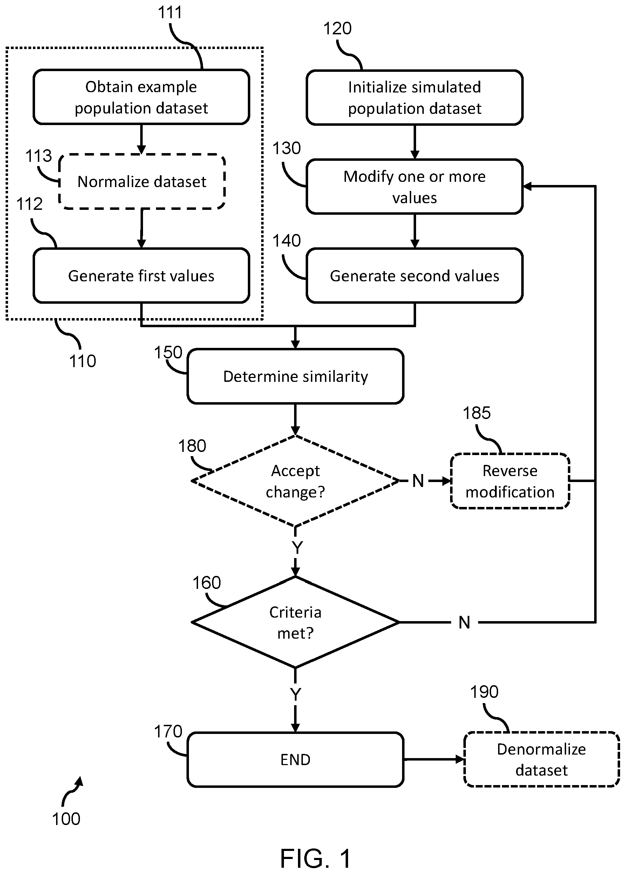

Fig. 1 is a flowchart illustrating a method 100 according to an embodiment. The method 100 may be performed by a processing system.

The method 100 comprises a step 110 of obtaining one or more first values. Each first value being a value of a statistical property of an example population dataset. The example population dataset contains example values for a plurality of subject parameters for a plurality of example medical subjects.

Thus, step 110 comprises obtaining statistical information of an example population dataset. The example population dataset represents real or ground-truth values of a real population.

In some examples, step 110 comprises obtaining the one or more first values from a data storage system, remote processing system or the like. Thus, the one or more first values may have been previously generated, e.g., at a remote processing system. This advantageously avoids the processing system performing method 100 from directly accessing the example population dataset, avoiding any concerns or considerations about maintaining privacy/anonymity of the example population dataset.

In other examples, step 110 is performed by performing a step 111 of obtaining the example population dataset; and a step 112 processing the example population dataset using at least one statistical analysis technique to produce the one or more first values. Example statistical analysis techniques will be provided later in this disclosure.

The method 100 also comprises a step 120 of defining a simulated population dataset containing simulated values for the plurality of subject parameters for a plurality of simulated medical subjects. The number of simulated medical subject may be greater than the number of example medical subjects.

The defined simulated population dataset may have been previously defined or determined. This can allow, for instance, a policy or user to define the size and structure of the simulated population dataset.

Alternatively, step 120 may be performed, for example, by creating a population dataset comprising a plurality of entries (the number of which can be predetermined, randomized or pseudo-randomized), each entry representing a different simulated subject. Each entry may comprise a random or pseudo-random value for the plurality of subject parameters represented by the example population dataset. For instance, step 120 may comprise generating a random or pseudorandom value for each of the simulated values, e.g., such that the distribution of values throughout the simulated population dataset is uniform. Another option is to randomly or pseudorandomly draw from a non-uniform distribution of potential values for the simulated values, e.g., a normal/Gaussian distribution, a log-normal distribution, a Weibull distribution and so on.

If the example population dataset contains values for X parameters of Y example medical subjects, then the simulated population dataset may contain random or pseudorandom values for X parameters of Z simulated medical subjects. Z may be any suitable value, e.g., equal to Y, user-defined or a random/pseudo-random value. In particular, the value of Z may be greater than Y.

In some examples, if performed, the step of generating a random or pseudorandom value for each of the simulated values is configured such that the distribution of values throughout the simulated population dataset is uniform.

Steps 110 and 120 may be performed sequentially or concurrently.

The example population dataset may contain a plurality of first data entries, each first data entry representing a different example medical subject and comprising a plurality of first data fields that each store a value for a different one of a plurality of subject parameters. The simulated population dataset may similarly contain a plurality of second data entries, each second data entry representing a different simulated medical subject and comprising a plurality of second data fields that each store a simulated value for a different one of the plurality of subject parameters.

The method then moves to an iterative process comprising a modification step 130 and a statistical analysis step 140.

The modification step 130 comprises modifying at least some of the simulated values in the simulated population dataset.

This may, for instance, be performed by selecting a plurality of simulated values using a random or pseudorandom selection process; and modifying the selected values. The total number of selected simulated values may be predetermined (e.g., a predetermined percentage or fraction) or random/pseudorandom. As an example, in preferable examples, no fewer than 1% of all simulated values are selected for modification, e.g., no fewer than 2% of all simulated values, e.g., no fewer than 5% of all simulated values, e.g., no fewer than 10% of all simulated values.

The modifying of the at least some of the simulated values, e.g., the selected values, may comprise perturbing the selected values. This may, for instance, comprise modifying the selected values to change by a random or pseudo-random amount (e.g., clipped to a maximum/minimum allowable value where relevant). Alternatively, the modifying may comprise modifying the selected values by a predetermined or random/pseudorandom percentage or fraction (e.g., up to a maximum size or amount). The precise mechanism for modifying the selected values is not essential for achieving the underlying inventive concept.

In some examples, the maximum size of the modifications is defined responsive to a user input. This can, for instance, allow the individual to control the amount of modification.

In some examples, the maximum size of the modifications reduces with increased number of iterations. Thus, the step of modifying at least some of the simulated values comprises performing larger modifications to the at least some simulated values during temporally earlier iterations of the iterative process than during temporally later iterations of the iterative process. This acts as a cooling down function for the iterative process, to ensure or achieve enhanced convergence.

The statistical analysis step 140 comprises processing the simulated population dataset using at least one statistical analysis technique to produce or determine one or more second values.

Each second value is the value of a different statistical property of the simulated population dataset, wherein each second value represents a value of a same statistical property as a corresponding first value. Thus, each second value (for the simulated population dataset) corresponds to a first value (for the example population dataset) and represents a same statistical property or measure.

The same at least one statistical analysis technique may be used to process the simulated population dataset (to produce the second value(s)) as was used to process the example population dataset (to produce the first value(s)). This ensures that the first and second values appropriately correspond to one another.

The iterative process of steps 130 and 140 is repeated until a similarity between the one or more first values and the one or more second values meets one or more predetermined criteria.

The similarity can be determined using a similarity determination step 150, which determines one or more measures of similarity and/or dissimilarity/difference between the first value(s) and the second value(s). A determination step 160 may determine whether or not the one or more measures of similarity or difference meet the predetermined criterion/criteria. The similarity determination step thereby quantifies the similarity/difference between the simulated population dataset (the generated data) and the example population dataset (the real data).

A measure of similarity and a measure of difference are considered to be semantically identical in the context of the present disclosure. Suitable examples will be provided later in this disclosure.

Responsive to the predetermined criterion/criteria being met, the iterative process ends in a step 170. Otherwise, the iterative process is repeated, i.e., the method repeats step 130.

The predetermined criterion/criteria may depend upon the determination of the difference between the first value(s) and the second value(s), e.g., the form of the measure(s) of similarity or difference.

The one or more predetermined criteria may include a criterion that a measure of similarity meets predetermined threshold conditions indicating that the one or more first values and the one or more second values are similar to one another. As an example, the one or more predetermined criteria may contain a criterion that a measure of similarity, e.g., determined in step 150, is greater than a predetermined value (or that a measure of difference is below a predetermined value). This ensures that the statistical properties of the simulated population dataset are sufficiently similar to the statistical properties of the example population dataset.

As another example, the one or more predetermined criteria may contain a criterion that the measure of similarity (or measure of difference) has not changed by more than a predetermined amount during a preceding predetermined period of time or number of iterations. This approach helps avoid the iterative process from continually looping, e.g., if a local minimum has been reached.

Other suitable criteria for terminating the iterative process responsive to a similarity between the one or more first values and the one or more second values will be apparent to the skilled person.

One or more alternative termination procedures may also be employed to control termination of the iterative process. For instance, the iterative process of steps 130 and 140 may be terminated responsive to a predetermined period of time elapsing since beginning the iterative process and/or a number of iterations of the iterative process exceeding a predetermined iteration number. These examples prevent the iterative process from looping endlessly when no convergence is likely, e.g., to avoid or reduce power wastage.

The iterative process of method 100 may include a selective modification process, in which the modifications performed in step 130 are only accepted if the modification helps to converge the first value(s) and second value(s).

In particular, the method 100 may comprise a determination step 180 which determines whether or not to accept the modification of step 130 responsive to one of the one or more measures of similarity/difference .

In particular, responsive to the measure of similarity meeting predetermined threshold conditions (e.g., reaching or breaching a predetermined similarity value), the modification to the at least some of the simulated values in the simulated population dataset is accepted, and the method moves to determination step 160.

Responsive to the measure of similarity failing to meet predetermined threshold conditions (e.g., not reaching or breaching the predetermined similarity value), the modification to the at least some of the simulated values in the simulated population dataset may be discarded or rejected (i.e., the modification is reversed) in a step 185.

The predetermined similarity value may be dependent upon the similarity value determined in a previous iteration of the iterative process (labelled the previous similarity value). For instance, the predetermined similarity value sₚ may be equal to the previous similarity value or a scalar multiple of the previous similarity value (e.g., 0.9sₚ or 0.95sₚ). As another example, the predetermined similarity value may be to the similarity value ± a predetermined bias value.

Using a scalar multiple or biased version of the previous similarity value allows for the iterative process to deliberately make mistakes, i.e., taking perturbations that show a worse fit, to enable escaping local optima. This may improve the fitting of the statistical properties of the simulated population dataset to those of the example population dataset.

Preferably, the example population dataset and the simulated population dataset are both normalized datasets. In the context of the present disclosure, a normalized dataset is a dataset that has undergone a normalization function such that all values lie within a same range or interval (e.g., between 0 and 1). This facilitates ease of initializing the values in the simulated population dataset as well as modifying/perturbing the simulated values. This approach also allows the values of statistical properties to be more accurately compared, as they are on a common scale.

The normalization function may, for instance, comprise performing a min-max correction, such that the minimum and maximum values for each subject values are scaled to predefined minimum and maximum values. For instance, the minimum value may be set to 0 and the maximum value may be set to 1. Other values are appropriately scaled to lie between the modified minimum and maximum values, e.g., scaled to lie within a predefined range or interval of [0,1].

It will be appreciated that other example predefined ranges or intervals could be used in alternative embodiments, e.g., an interval of [0,10], [1,10], [0,100] or [1,100]. The precise min-max values are immaterial to the execution of the method.

However, various other approaches for performing a normalization function on a dataset, which could be employed in the present disclosure, are well established in the art, such as those suggested in Eesa, Adel S., and Wahab Kh Arabo. "A normalization methods for backpropagation: a comparative study." Science Journal of University of Zakho 5.4 (2017): 319-323. This same document also discloses an approach for performing min-max correction. Kim, Daehyon. "Normalization methods for input and output vectors in backpropagation neural networks." International journal of computer mathematics 71.2 (1999): 161-171 also provides examples of how to normalize a dataset, which could be adopted for use with the present disclosure.

In some examples, the example population dataset is produced by performing a normalization function on an initial example population dataset. The initial population dataset may, for instance, contain values for subject parameters that correspond to expected values for each subject parameter (e.g., lie within an expected range for said subject parameter).

The normalization process may form part of step 110, if the example population dataset is directly obtained and processed to generate the one or more first values.

In particular, normalization may be performed in an optional normalization step 113 on an obtained example population dataset. In another example, the example population dataset obtained in step 111 may already be normalized, e.g., by an external/different processing system to that performing method 100.

The computer-implemented method 100 may further comprise, after the iterative process is terminated (in step 170), a step 190 of performing the inverse of the normalization function on the simulated population dataset. This produces a simulated population dataset that contains values lying within the same range as the example population dataset and thereby exhibits expected or plausible values for each subject parameter.

Approaches for inversing a normalization process will be apparent to the skilled person, and are not disclosed for the sake of conciseness.

In some examples, the plurality of subject parameters comprises at least one binary parameter and/or at least one categorical parameter. The method 100 may be appropriately adapted to take account of these value types.

For instance, modifying at least some of the simulated values (in step 130) may comprise modifying switching a value associated with a binary parameter (e.g., from 0 to 1 or vice versa) if a value for a binary parameter is modified/selected for modification. Similarly, modifying at least some of the simulated values may comprise changing the value of a categorical parameter to indicate a different a category (similarly, if a value for a categorical value is modified or selected for modification). Of course, the precise modification may depend upon the type (e.g., binary, categorical or numeric) of parameter for the value(s) that is/are to be modified, as would be appreciated by the skilled person.

As previously explained, in some examples, each value of the example/simulated population dataset has been normalized to lie within a predefined range. For a binary parameter, the binary choice can be readily indicated by setting the maximum and minimum value of this range to represent a particular binary option. For a categorical parameter, the category can be indicated by a particular value within the predefined range, the number of potential values for the categorical value being defined by the number of potential categories for the categorical value/parameter. Thus, each binary/categorical value can be represented by a particular numeric value.

Thus, for binary/categorical parameter, there may be a restricted set of allowable numeric values (lying within the predefined range) that each represent a different binary or categorical value. For a categorical parameter, the allowable numeric values may be evenly spread or distributed throughout the predefined range. For a binary parameter, the allowable numeric values may be a maximum and minimum value within the predefined range.

The modification step 130 may be tweaked/adapted, for binary/categorical parameters, such that the modified value remains a binary or categorical value. If a binary/categorical value is represented by a numeric value, step 130 could be performed for that value by modifying the numeric value using a previously described approach (e.g., scaling or biasing by a random/pseudorandom value) before performing a thresholding process to assign the modified numeric value to allowable numeric values representing a binary/categorical value.

For instance, if a modified parameter is a binary parameter, then the numeric value for the binary parameter may be modified using a numeric modification process. A thresholding process may be used to reassign the modified numeric value to a maximum or minimum value of the predefined range to ensure that the binary value retains its binary nature. For instance, if the modified numeric value for the binary parameter is greater than or equal to N_{T} (where N_{T} is equal to the midpoint between the maximum and minimum value for the predefined range), then the numeric value may be set to be equal to the maximum value for the predefined range - otherwise, the numeric value may be set to be equal to the minimum value for the predefined range.

This principle can be readily extended to handle categorical data formats by performing a multi-level thresholding, the number of levels being dependent upon the number of potential categories in the categorical parameter.

It has previously been described how one or more first values and one or more second values are obtained/generated and used to control the iterative process. Each first/second value is a value of a statistical property of the corresponding population dataset. Each second value provides a value of a same statistical property as a corresponding first value.

In preferred examples, the one or more first values comprises a co-variance matrix of the example population dataset; and the one or more second values comprises a co-variance matrix of the simulated population dataset. Thus, each first and second value represents a value of a co-variance matrix of its corresponding population dataset.

Thus, a covariance matrix can be used as the key statistical property of the example population dataset to replicate with the simulated population dataset.

A co-variance matrix quantifies the relationship between different subject parameters in a population dataset, which has been recognized as being the most important characteristic or feature of a population dataset for the purposes of training a machine-learning method (which is typically trained to learn a causal relationship between different parameters) and any other form of data analysis. In this way, the simulated population dataset retains or simulates the most important characteristic of the example population dataset for accurate analysis of later medical subjects.

A measure of similarity and/or difference can be determined (e.g. for step 150) by, for instance, determining the sum or average of absolute differences between the covariance matrix of the example population dataset and the simulated population dataset. This provides a single, representative value of the statistical similarity/difference between the two datasets.

In particular, each first value may represent a value in the covariance matrix for the example population dataset (between a covariance between variable X and variable Y) and each second value may represent a value in the covariance matrix for the simulated population dataset that corresponds to one of the first values. A difference between each second value and its corresponding first value may be determined. A sum or average of these determined differences can then be determined as the measure of similarity.

However, one or more values for other forms of statistical information or properties can be generated instead of, or in addition to, a co-variance matrix and used to determine the measure of similarity. Thus, one or more other statistical properties of a population dataset may also/otherwise be used.

Another example of a suitable statistical property of a population dataset is a measure of skewness. Accordingly, the termination of the iterative process may be responsive to at least a difference in skewness or skewnesses between the example population dataset and the simulated population dataset.

For instance, for the example population dataset and the simulated population dataset, a measure of skewness may be generated for each subject parameter shared by the population datasets. A difference between the sum or average of the measures of skewness may be determined as a measure of similarity.

In another example, a difference between a multi-variate skewness of the example population dataset and the simulated population dataset may be determined as a measure of similarity.

Another example of a suitable statistical property of a population dataset is a measure of kurtosis. Accordingly, the termination of the iterative process may be responsive to at least a difference in kurtosis between the example population dataset and the simulated population dataset.

For instance, for the example population dataset and the simulated population dataset, a measure of kurtosis may be generated for each subject parameter shared by the population datasets. A difference between the sum or average of the measures of kurtosis may be determined as a measure of similarity.

In another example, a difference between a multi-variate kurtosis of the example population dataset and the simulated population dataset may be determined as a measure of similarity.

Approaches for generating measures of skewness and kurtosis are established in the art. Cain, Meghan K., Zhiyong Zhang, and Ke-Hai Yuan. "Univariate and multivariate skewness and kurtosis for measuring nonnormality: Prevalence, influence and estimation." Behavior research methods 49.5 (2017): 1716-1735 provides some example approaches for determining such measures.

Various other multi-variate statistical properties could be used to advantage in other embodiments of the inventive concept. A number of these statistical properties are set out in Tabachnick, Barbara G., Linda S. Fidell, and Jodie B. Ullman. Using multivariate statistics. Vol. 5. Boston, MA: pearson, 2007, any of which could be employed in different embodiments of the present disclosure.

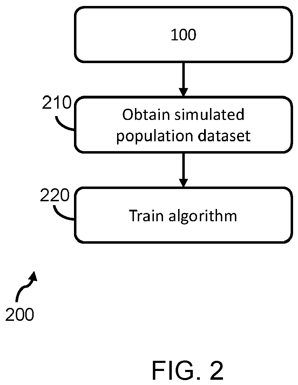

Fig. 2 illustrates a method 200 according to an embodiment. The method 200 is for training a machine-learning method to perform a predictive task.

A machine-learning algorithm is any self-training algorithm that processes input data in order to produce or predict output data.

Suitable machine-learning algorithms will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

The method 200 comprises a step 210 of obtaining a population dataset include a simulated population dataset generated by performing the method 100 previously described. The obtained population dataset may optionally also comprise an example population dataset (i.e., a non-simulated population dataset), although this is less preferred for reasons of anonymity/privacy.

The method 200 also comprises a step 220 of training the machine-learning method to perform the predictive task using the population dataset as ground truth data.

Methods of training a machine-learning algorithm are well known.

Typically, such methods comprise using a population dataset to train the machine-learning algorithm. The population dataset defines training input data entries and corresponding training output data entries. The input data entries correspond to input parameters for the machine-learning algorithm and the output data entries correspond to desired output parameters for the machine-learning algorithm.

An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

Other approaches for training a machine-learning algorithm using a population dataset as ground truth data are well known in the art of artificial intelligence, and are not described for the sake of conciseness. An overview of a number of approaches is described by Alzubi, Jafar, Anand Nayyar, and Akshi Kumar. "Machine learning from theory to algorithms: an overview." Journal of physics: conference series. Vol. 1142. No. 1. IOP Publishing, 2018 or Bhavsar, Hetal, and Amit Ganatra. "A comparative study of training algorithms for supervised machine learning." International Journal of Soft Computing and Engineering (IJSCE) 2.4 (2012): 2231-2307.

In this way, the simulated population dataset is used in the training of a machine-learning method.

In any above described embodiment, it is not essential that the simulated population dataset contain values for the same number of subject parameters as the example population dataset.

In particular examples, the number of subject parameters represented in the simulated population dataset may be less than or equal to the number of subject parameters represented in the example population dataset. This can, for instance, allow the simulated population dataset to be generated for only a subset (i.e., not all) of the subject parameters, e.g., those of interest for training a machine-learning algorithm and/or data analysis). Thus, in some examples, the number of subject parameters represented in the simulated population dataset is less than the number of subject parameters represented in the example population dataset.

However, for improved performance, the one or more first values and/or the one or more second values may provide information on one or more statistical properties of only those values for subject parameters shared by the example and population datasets.

Thus, the simulated population dataset may effectively be a simulated version of only a part of a (larger) example population dataset, e.g., to provide information on only a subset of the subject parameters represented in the example population dataset.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium storing the computer program.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims.

The mere fact that certain measures are recited in mutually different dependent claims does not indicate that a combination of these measures cannot be used to advantage.

If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A computer-implemented method for generating a simulated population dataset, the computer-implemented method comprising:
obtaining one or more first values, each first value being a value of a statistical property of an example population dataset, the example population dataset containing example values for a plurality of subject parameters for a plurality of example medical subjects;
defining a simulated population dataset containing simulated values for the plurality of subject parameters for a plurality of simulated medical subjects;
performing an iterative process comprising:
modifying at least some of the simulated values in the simulated population dataset; and
determining one or more second values by processing the simulated population dataset using at least one statistical analysis technique, each second value being the value of a statistical property of the simulated population dataset, wherein each second value represents a value of a same statistical property as a corresponding first value;
wherein the iterative process is terminated responsive to a similarity between the one or more first values and the one or more second values meeting one or more predetermined criteria.

2. The computer-implemented method of claim 1, wherein:
the one or more first values comprises a co-variance matrix of the example population dataset; and
the one or more second values comprises a co-variance matrix of the simulated population dataset.

3. The computer-implemented method of any of claims 1 or 2, wherein the step of defining a simulated population dataset comprises generating a random or pseudorandom value for each of the simulated values.

4. The computer-implemented method of any of claims 1 to 3, wherein the example population dataset and the simulated population dataset are both normalized datasets.

5. The computer-implemented method of claim 4, wherein:
the example population dataset is produced by performing a normalization function on an initial example population dataset; and
the computer-implemented method further comprises, after the iterative process is terminated, performing the inverse of the normalization function on the simulated population dataset.

6. The computer-implemented method of any of claims 1 to 5, wherein the plurality of subject parameters comprises at least one binary parameter and/or at least one categorical parameter.

7. The computer-implemented method of any of claims 1 to 6, wherein the step of modifying at least some of the simulated values comprises:
selecting a plurality of simulated values using a random or pseudorandom selection process; and
modifying the selected values.

8. The computer-implemented method of any of claims 1 to 7, wherein the step of modifying at least some of the simulated values comprises performing larger modifications to the at least some simulated values during temporally earlier iterations of the iterative process than during temporally later iterations of the iterative process.

9. The computer-implemented method of any of claims 1 to 8, wherein the iterative process further comprises:
processing the one or more first values and the one or more second values to determine a measure of similarity between the one or more first values and the one or more second values;
responsive to the measure of similarity meeting predetermined threshold conditions, accepting the modification to the at least some of the simulated values in the simulated population dataset; and
responsive to the measure of similarity failing to meet the predetermined threshold conditions, discarding or rejecting the modification to the at least some of the simulated values in the simulated population dataset.

10. The computer-implemented method of any of claims 1 to 9, wherein:
the one or more first values comprises a measure of skewness and/or a measure of kurtosis of the example population dataset; and
the one or more second values comprises a measure of skewness and/or a measure of kurtosis of the simulated population dataset.

11. The computer-implemented method of any of claims 1 to 10, wherein the one or more predetermined criteria include a criterion that a measure of similarity between the one or more first values and the one or more second values meets predetermined threshold conditions indicating that the one or more first values and the one or more second values are similar to one another.

12. The computer-implemented method of any of claims 1 to 11, wherein the step of obtaining one or more first values comprises:
obtaining the example population dataset; and
processing the example population dataset using the at least one statistical analysis technique to produce the one or more first values.

13. A computer-implemented method for training a machine-learning method to perform a predictive task, the computer-implemented method comprising:
obtaining a population dataset comprising a simulated population dataset generated by performing the method of any of claims 1 to 12; and
training the machine-learning method to perform the predictive task using the population dataset as ground truth data.

14. A computer program product comprising computer program code means which, when executed on a computing device having a processing system, cause the processing system to perform all of the steps of the method according to any of claims 1 to 13.

15. A processing system for generating a simulated population dataset, the processing system being configured to:
obtain one or more first values, each first value being a value of a statistical property of an example population dataset, the example population dataset containing example values for a plurality of subject parameters for a plurality of example medical subjects;
define a simulated population dataset containing simulated values for the plurality of subject parameters for a plurality of simulated medical subjects; and
perform an iterative process comprising:
modifying at least some of the simulated values in the simulated population dataset; and
determining one or more second values by processing the simulated population dataset, each second value being the value of a statistical property of the simulated population dataset, wherein each second value represents a value of a same statistical property as a corresponding first value,
wherein the iterative process is terminated responsive to a similarity between the one or more first values and the one or more second values meeting one or more predetermined criteria.
